# EUROPEAN PATENT SPECIFICATION

(11) **EP 3 643 358 B1**
(45) Date of publication and mention of the grant of the patent: **02.06.2021**
(21) Application number: 19190996.9
(22) Date of filing: 16.09.2016
(51) Int. Cl.: A61N 1/378, A61N 1/375, A61N 1/362

(54) **ASSEMBLY TECHINIQUES FOR SINTERED ANODES AND CATHODES**
MONTAGEVERFAHREN FÜR GESINTERTE ANODEN UND KATHODEN
TECHNIQUES D'ASSEMBLAGE POUR ANODES ET CATHODES FRITTÉES

(30) Priority: 16.09.2015 US 201562219278 P
(43) Date of publication of application: 29.04.2020
(62) Divisional of application: 16770652.2
(73) Proprietor: Cardiac Pacemakers, Inc., St. Paul, MN 55112-5798 (US)
(72) Inventor: SHERWOOD, Gregory J., North Oaks, MN 55127 (US); KUHN, Peter Jay, Crystal, MN 55422 (US); OLSON, Jaymes, Lino Lakes, MN 55014 (US); ANDERSON, Matthew, Arden Hills, MN 55112 (US); WAYTASHEK, Brian V., Lino Lakes, MN 55014 (US); FLOETER, Aaron, Waconia, MN 55387 (US)
(74) Representative: Peterreins Schley

(56) References cited:
- US-A1- 2011 152 958
- US-A1- 2011 152 959
- US-A1- 2011 152 960
- US-A1- 2011 317 370
- US-A1- 2013 041 420

## Description

### TECHNICAL FIELD

This document relates generally to energy storage and particularly to sintered electrodes to store energy in an implantable medical device.

### BACKGROUND

Electrical stimulation therapy has been found to benefit some patients. For example, some patients suffer from an irregular heartbeat or arrhythmia and may benefit from application of electrical stimulation to the heart. Some patients suffer from a particular type of arrhythmia called a fibrillation. Fibrillations may affect different regions of the heart, such as the atria or the ventricles. When a fibrillation occurs in the ventricles, the heart's ability' to pump blood is dramatically reduced, putting the patient at risk of harm. It has been found that applying an electrical stimulation to the patient can effectively treat patients suffering disorders such as from fibrillation by restoring a regular heartbeat.

Because disorders such as fibrillations can happen at any time, it is helpful to have a device that is easily accessible to treat them. In some cases, it is helpful if that device is portable or implantable. In developing a device that is portable or implantable, it is helpful to have access to components that are compact and lightweight and that can perform to desired specifications.

US 2011/152958 A1, US 2013/041420, US 2011/317370 A1, US 2011/152959 A1, and US 2011/152960 A1 describe capacitor arrangements including anodes and cathodes for implantable medical devices.

### SUMMARY

The invention is defined in the appended claims. Aspects, embodiments and examples disclosed herein which do not fall within the scope of the appended claims do not form part of the invention, and are merely provided for illustrative purposes.

A first general aspect of the invention relates to a method of assembling a capacitor includes sintering cathode material directly to an inside surface of a capacitor case, the cathode material forming one or more sintered cathodes having a shape; and placing a sintered anode over or around the sintered cathodes, the sintered anode having one or more mating portions that match the shape of the one or more sintered cathodes such that the mating portions matingly receive the sintered cathodes.

In example 2, the subject matter of the first general aspect can optionally include the mating portions of the sintered anode being shaped like a fin and the sintered cathode having a fin shape.

In example 3, the subject matter of the first general aspect can optionally include the sintered cathode having a cylindrical shape and the mating portions of the sintered anode including rounded cut-outs at one or more comers of the sintered anode.

In example 4, the subject matter of the first general aspect can optionally include, the mating portions being conical and the sintered cathode being conical.

In example 5, the subject matter of any of the first general aspect or of examples 2-4 can optionally include the cathode including a bed of nails structure.

In example 6, the subject matter of any the first general aspect or of examples 2-5 can optionally include a separator applied to the cathode, wherein the separator includes a high dielectric polymer directly applied to an outer surface of the sintered cathode.

A second general aspect of the invention relates to a method of assembling a capacitor includes sintering cathode material directly to a PCB within a capacitor case, the cathode material forming one or more sintered cathodes having a shape; and placing a sintered anode over the sintered cathodes , the sintered anode having one or more mating portions that match the shape of the one or more sintered cathodes such that the mating portions matingly receive the sintered cathodes.

In example 7, the subject matter of the second general aspect can optionally include the mating portions of the sintered anode being shaped like a fin and the sintered cathode having a fin shape.

In example 8, the subject matter of claim 7 can optionally include the sintered cathode having a cylindrical shape and the mating portions of the sintered anode including rounded cut-outs at one or more comers of the sintered anode.

In example 9, the subject matter of the second general aspect can optionally include the mating portions being conical and the sintered cathode being conical.

Further general aspects of the present invention relate to the apparatus as set out in claims 13 and 14.

### BRIEF DESCRIPTION OF THE DRAWINGS

The drawings illustrate generally, by way of example, various embodiments discussed in the present document. The drawings are for illustrative purposes only and may not be to scale.
FIG. 1 shows a schematic representation of a medical system including a sintered capacitor, in accordance with one embodiment.
FIG. 2 shows an implanted medical system including a sintered capacitor, in accordance with one embodiment.
FIG. 3 shows a capacitor, in accordance with one embodiment.
FIG. 4 shows a detail of the capacitor of FIG. 3, in accordance with one embodiment.
FIG. 5 shows a detail of the capacitor of FIG. 3, in accordance with one embodiment.
FIG. 6 shows a detail of the capacitor of FIG. 3, in accordance with one embodiment.
FIG. 7 shows a detail of the capacitor of FIG. 3, in accordance with one embodiment.
FIG. 8 shows a capacitor, in accordance with one embodiment.
FIG. 9 shows a capacitor, in accordance with one embodiment.
FIG. 10 shows a capacitor, in accordance with one embodiment.
FIG. 11 shows a capacitor, in accordance with one embodiment.
FIG 12 shows an anode, in accordance with one embodiment.
FIG. 13 shows a capacitor, in accordance with one embodiment.
FIG. 14 shows a capacitor, in accordance with one embodiment.

### DETAILED DESCRIPTION

The following detailed description of the present invention refers to subject matter in the accompanying drawings which show, by way of illustration, specific aspects and embodiments in which the present subject matter may be practiced. These embodiments are described in sufficient detail to enable those skilled in the art to practice the present subject matter. References to "an", "one", or "various" embodiments in this disclosure are not necessarily to the same embodiment, and such references contemplate more than one embodiment. The following detailed description is, therefore, not to be taken in a limiting sense, and the scope is defined only by the appended claims, along with the full scope of legal equivalents to which such claims are entitled.

This document concerns sintered electrodes for use in an electrical energy storage device. Specific examples include sintered anodes formed of aluminum or its alloys. Certain examples are for use in aluminum electrolytic capacitors. Additional benefits stem from an increased surface area that is a product of sintering.

Sintering results in many interstices (i.e., spaces) between grains of the electrode. Sintered electrodes resemble crushed grains with interstices between the grains. The interstices are filled with electrolyte, thereby increasing capacitance per unit of volume, as capacitance is proportional to a surface area exposed to electrolyte. An electrode with such interstices offers improved lateral or parallel movement of electrons in relation to a major surface of a flat electrode layer, as etched electrodes restrict lateral movement because the etchings result in voids that are typically perpendicular to the major surface of the flat layer. Accordingly, some examples have a lower ESR (equivalent series resistance) compared to etched foils due to this enhance ionic flow.

Overall, an energy storage device using the sintered electrodes described here is well suited for use in an implantable medical device such as a defibrillator. Because sintering can produce a variety of shapes, sintered electrodes can be used to create energy storage devices such as capacitors that have custom shapes versus simple rolled cylinders or a prism having a parallelogram as its base. Further, manufacturing efficiency is improved, by easing the steps and parts in manufacturing a capacitor and by reducing waste. The interstices are very small, making the electrodes rigid and able to withstand handling by a machine or assembly personnel. These electrodes demonstrate an improved energy density over etched electrodes and are therefore useful to make smaller implantable devices that are able to deliver an amount of energy for a particular therapy.

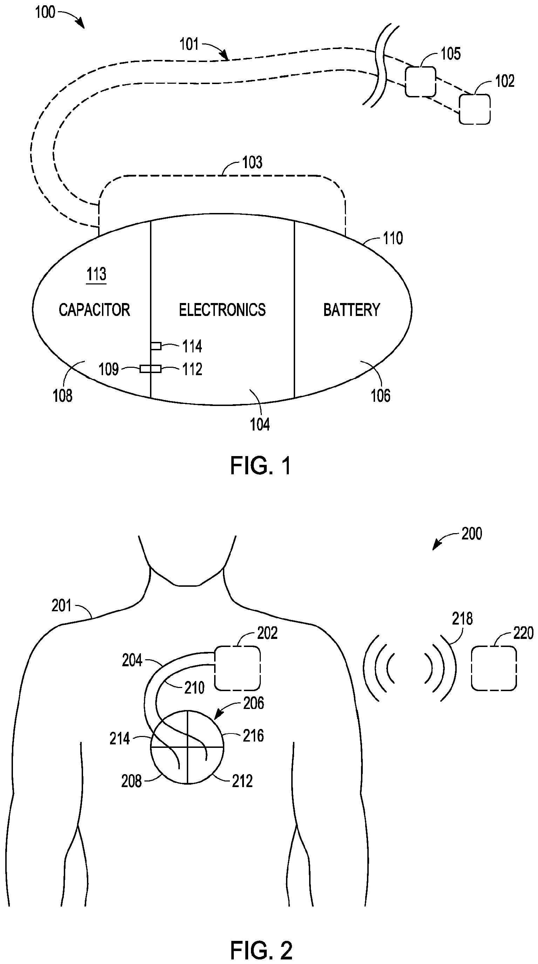

FIG. 1 is a schematic of a medical system 100 including a sintered capacitor, according to some embodiments. The medical system 100 represents any number of systems to provide therapeutic stimulus, such as to a heart. Examples of medical systems include, but are not limited to, implantable pacemakers, implantable defibrillators, implantable nerve stimulation devices and devices that provide stimulation from outside the body, including, but not limited to, external defibrillators.

Electronics 104 are to monitor the patient, such as by monitoring a sensor 105, and to monitor and control activity within the system 100. In some examples, the electronics 104 are to monitor a patient, diagnose a condition to be treated such as an arrhythmia, and control delivery of a stimulation pulse of energy to the patient. The electronics 104 can be powered wirelessly using an inductor. Alternatively, the electronics 104 can be powered by a battery 106. In some examples, electronics 104 are to direct small therapeutic bursts of energy to a patient from the battery 106.

For therapies, such as defibrillation, that use energy discharge rates exceeding what battery 106 is able to provide, a capacitor 108 is used. Energy from the battery 106 is controlled by the electronics 104 to charge the capacitor 108. The capacitor 108 is controlled by the electronics 104 to discharge to a patient to treat the patient. In some examples, the capacitor 108 completely discharges to a patient, and in additional examples, the capacitor is switched on to provide therapeutic energy and switched off to truncate therapy delivery.

Some examples of a medical system 100 include an optional lead system 101. In certain instances, after implantation, the lead system 101 or a portion of the lead system 101 is in electrical communication with tissue to be stimulated. For example, some configurations of lead system 101 contact tissue with a stimulation electrode 102. The lead system 101 couples to other portions of the system 100 via a connection in a header 103. Examples of the system 101 use different numbers of stimulation electrodes and/or sensors in accordance with the needs of the therapy to be performed.

Additional examples function without a lead 101. Leadless examples can be positioned in contact with the tissue to be stimulated, or can be positioned proximal to tissue to shock the tissue to be stimulated through intermediary tissue. Leadless examples can be easier to implant and can be less expensive as they do not require the additional lead components. The housing 110 can be used as an electrode in leadless configurations.

In certain embodiments, the electronics 104 include an electronic cardiac rhythm management circuit coupled to the battery 106 and the capacitor 108 to discharge the capacitor 108 to provide a therapeutic pulse, such as a defibrillation pulse. In some examples, the system 100 includes an anode and a cathode sized to deliver a pulse of at least approximately 50 joules. Other configurations can deliver larger amounts of energy. Some configurations deliver less energy. In some examples, the energy level is predetermined to achieve a delivered energy level mandated by a governing body or standard associated with a geographic region, such as a European country. In an additional embodiment, the anode and cathode are sized to deliver a defibrillation pulse of at least approximately 60 joules. In some examples, this is the energy level is predetermined to achieve an energy level mandated by a governing body of another region, such as the United States. In some examples, electronics 104 are to control discharge of a defibrillation pulse so that the medical system 100 delivers only the energy mandated by the region in which the system 100 is used.

One characteristic of some sintered electrode examples is that at least one anode and a cathode have a DC capacitance that is approximately 23% greater than a AC capacitance for the at least one anode and the cathode of an etched capacitor that has 74.5 microfarads per cubic centimeter. In some examples, the at least one anode and the cathode have an AC capacitance of at least 96.7 microfarads per cubic centimeter at 445 total voltage. In some examples, this is comparable to an operating voltage of about 415 volts. This is a 30% improvement over an etched capacitor that has 74.5 microfarads per cubic centimeter. Total voltage is the voltage that allows 1 milliamp of leakage per square centimeter for an electrode. Some examples are aged to 415 volts.

In certain examples, the capacitor 108 includes a capacitor case 113 sealed to retain electrolyte. In some examples, the capacitor case 113 is welded. In some instances, the capacitor case 113 is hermetically sealed. In additional examples, the capacitor case 113 is sealed to retain electrolyte, but is sealed with a seal to allow flow of other matter, such as gaseous diatomic hydrogen or a helium molecule. Some of these examples use an epoxy seal. The capacitor further includes a conductor 109 coupled to one of the electrodes of the capacitor 108. The conductor 109 sealingly extends through the capacitor case to a first terminal 112 disposed on an exterior of the capacitor case 113. A second terminal 114 can be disposed on the exterior of the capacitor case 113 and in electrical communication with the other electrode of the capacitor 108. The first terminal 112 and the second terminal 114 are electrically isolated from one another.

A hermetically sealed device housing 110 is used to house components, such as the battery 106, the electronics 104, and the capacitor 108. Hermeticity is provided by welding components into the hermetically sealed device housing 110, in some examples. Other examples bond portions of the housing 110 together with an adhesive such as a resin based adhesive such as epoxy. Accordingly, some examples of the housing 110 include an epoxy sealed seam or port. Several materials can be used to form housing 110, including, but not limited to, titanium, stainless steel, nickel, a polymeric material, or combinations of these materials. In various examples, the housing 110 and the case 113 are biocompatible.

The capacitor 108 is improved by the present electrode technology in part because it can be made smaller and with less expense and a variety of shapes and configurations. The improvement provided by these electrodes is pertinent to any application where high-energy, high-voltage, or space-efficient capacitors are desirable, including, but not limited to, capacitors used for photographic flash equipment. The present subject matter extends to energy storage devices that benefit from high surface area sintered electrodes including, but not limited to, aluminum. The electrodes described here can be incorporated into cylindrical capacitors that are wound, in addition to stacked capacitors.

FIG. 2 is an implanted medical system 200, implanted in a patient 201, and including a sintered capacitor, according to some embodiments. The system includes a cardiac rhythm management device 202 coupled to a first lead 204 to extend through the heart 206 to the right ventricle 208 to stimulate at least the right ventricle 208. The system also includes a second lead 210 to extend through the heart 206 to the left ventricle 212. In various embodiments, one or both of the first lead 204 and the second lead 210 include electrodes to sense intrinsic heart signals and to stimulate the heart. The first lead 204 is in direct contact (e.g., touching) with the right atrium 214 and the right ventricle 208 to sense and/or stimulate both those tissue regions. The second lead 210 is in direct contact with the left atrium 216 and the left ventricle 212 to sense and/or stimulate both those tissue regions. The cardiac rhythm management device 202 uses the lead electrodes to deliver energy to the heart, either between electrodes on the leads or between one or more lead electrodes and the cardiac rhythm management device 202. In some examples, the cardiac rhythm management device 202 is programmable and wirelessly communicates 218 programming information with a programmer 220. In some examples, the programmer 220 wirelessly 218 charges an energy storage device of the cardiac rhythm management device 202.

The present system allows for different concepts for the design of high voltage aluminum electrolytic capacitors. As will be discussed the present system allows for reducing assembly time and cost by providing shapes that allow for ease of assembly with reduction of precise robotic assembly.

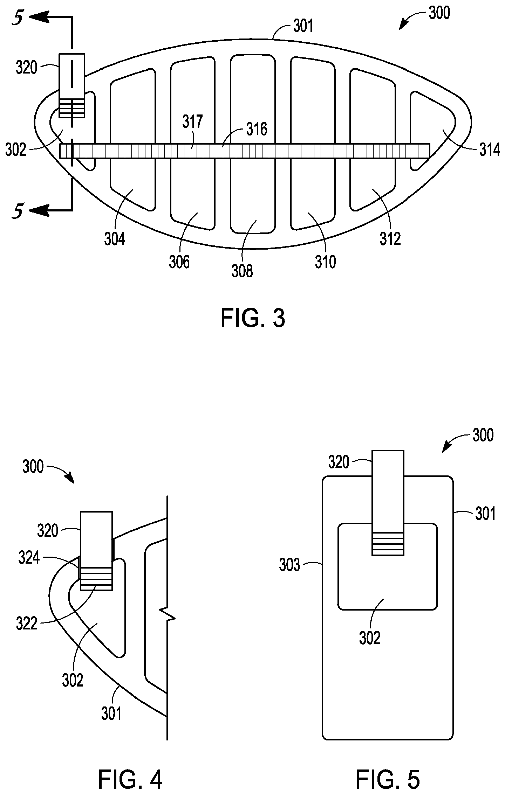

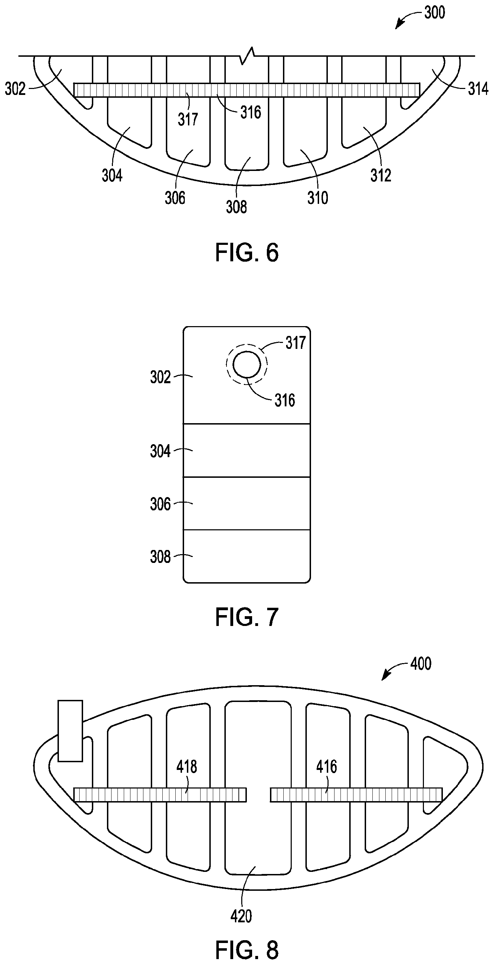

FIGs 3-7 show a capacitor 300, in accordance with one embodiment. FIG. 3 shows a top view of the capacitor 300. FIG. 4 shows a detail of a feedthrough 320 for the capacitor 300. FIG. 5 shows a side view of FIG. 4. FIG. 6 shows a detail of a connection post 316 for the capacitor 300. FIG. 7 is a side view of FIG. 6.

The capacitor 300 generally includes a capacitor case 301 which can be sealed to retain electrolyte and a plurality of sintered anodes 302-314. The anodes 302-314 each have a hole 317 at least partially through the sintered anode 302-314. An interconnect post such as a threaded interconnect post 316 can be placed through the holes 317 of the plurality of sintered anodes 302-314 to electrically couple the sintered anodes. In one example, each of the holes 317 can be a threaded hole, configured to match the thread of post 316. In an example, each of the anodes 302-314 are made of sintered aluminum and the threaded post 316 is made from aluminum. This system allows the interconnection to be made between the anodes with requiring the welding of tabs or other structures. A sintered cathode can be formed to cover the anodes with a separator there between, as discussed below.

FIGs 4 and 5 show further details of the feedthrough 320 for capacitor 300. In this example, anode 302 includes a second hole 322. A threaded feedthrough post 320 is connected to the anode at hole 322. In one example, hole 322 can be a threaded hole. The feedthrough post 320 extends through case 301 at an opening 324, where the post can be electrically insulated form the case 301. A cover 303 can be welded to case 310.

FIGs 6-7 show details of connection post 316 extending through holes 317 of the anodes 302-314.

FIG. 8 shows another interconnection configuration for the capacitor 300. In this example, two connection post 416 and 418, such as threaded connection posts are utilized. At least one anode 420 does not have a connection hole all the way through the anode, but instead has two partial holes two receive an end of post 418 and an end of post 416. In other embodiments, multiple holes and connection rods can be utilized, depending on need.

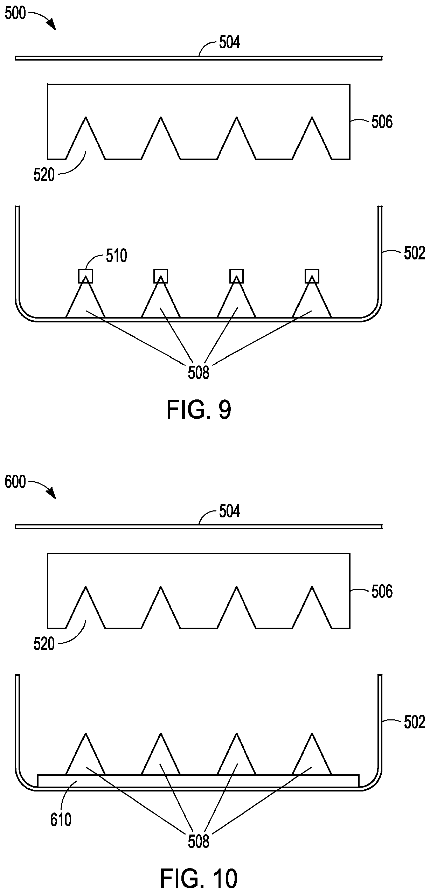

FIG. 9 shows a side view of a capacitor 500 in accordance with one embodiment. Capacitor 500 generally includes a case 502, a lid 504, an anode 506 and cathodes 508. Capacitor 500 allows for ease of manufacturing of capacitors since the case 502 itself is used as an assembly device.

For example, to assemble the capacitor 500, cathode material can be sintered directly to the inside surface of a capacitor case 502, the sintered cathode material forming one or more sintered cathodes 508 having a given shape. Then, the sintered anode 520 can be placed over or around the sintered cathodes 508. The sintered anode 506 can include one or more mating portions such as voids 520 that match the shape of the one or more sintered cathodes 508 such that the voids 520 matingly receive the sintered cathodes 508.

In one example, the mating portions of the sintered anode 506 can shaped like a fin and the sintered cathode 508 can have a fin shape. In one example, the void shape 520 can be conical and the sintered cathode 508 can be conical. In one example, the cathode 508 can include a bed of nails structure and a mating anode can be dropped over the bed of nails. Such shapes and structures allow for ease of assembly with lower complexity. In an example, a separator 510 can be applied to the cathode 508. For example, the separator 510 can include a high dielectric polymer directly applied to an outer surface of the sintered cathode.

As described above for FIG. 1, after assembling the cathodes and the anodes a conductor can be coupled to the sintered anode, with the conductor sealingly extending through the capacitor case to a terminal disposed on an exterior of the capacitor case. A second terminal can disposed on the exterior of the capacitor case and in electrical communication with the sintered cathode, with the terminal and the second terminal electrically isolated from one another.

FIG. 10 shows a capacitor 600, similar to capacitor 500, discussed above. Here the capacitor 600 includes sintering the cathode material directly to a PCB 610 that is within the capacitor case 502. The cathode material forms one or more sintered cathodes 508 having a desired shape. The sintered anode 506 can be placed over the sintered cathodes 508 such that the void spaces 520 match the shape of the one or more sintered cathodes 508 such that the voids matingly receive the sintered cathodes 508. Again, a separator such as discussed above can be placed on the cathode 508

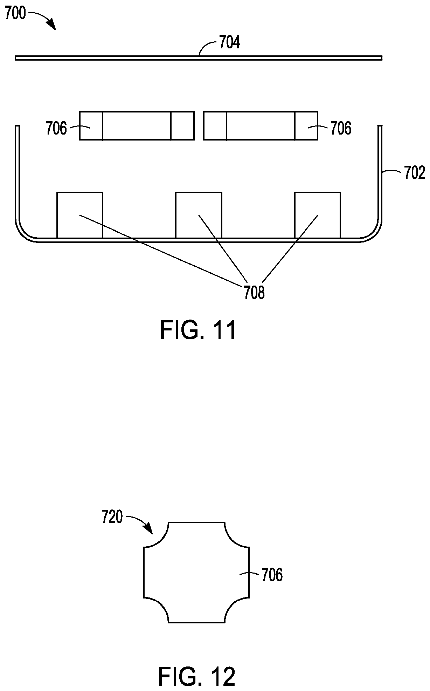

FIG. 11 shows an example of a capacitor 700 configured to allow for ease of manufacturing of capacitors since the case 702 itself is used as an assembly device. Here the capacitor includes the case 702, a lid 704, sintered anodes 706 and sintered cathodes 708. FIG. 12 shows a top view of the anode 706. The anodes 706 include a mating portion, such as a void space 720 comprising a rounded cut-out at one or more corners of the sintered anode 706. The cathodes 708 can have a cylindrical shape and can include a high dielectric polymer directly applied to an outer surface of the sintered cathode 706. To assemble, the cylindrical cathodes 708 are sintered directly to the surface of the case 702 and the anodes 706 are placed within the case like puzzle pieces such that the void spaces 720 matingly receive the sintered cathodes 708.

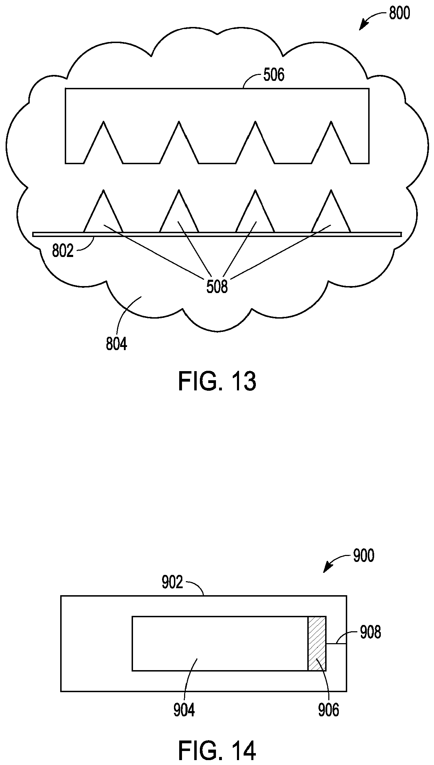

FIG. 13 shows a capacitor 700 including the anode 506 with mating portions including the void spaces 520 and the cathode 508. Here, the capacitor 700 is formed by sintering cathode material onto an aluminum foil 802, the cathode material forming one or more sintered cathodes 508 having a desired shape. The anode 506 is placed over the cathode 508, as discussed above. In this example, a polymer coating 804 is applied over the sintered anode 506, the sintered cathode 508 and the aluminum foil 802.

FIG. 14 shows a capacitor 900 including a case 902 and a sintered anode 904 disposed within the case 902. The sintered anode includes a masked, unsintered portion 906 configured for attaching the sintered anode 904 to a conductor 908, by welding for example. In other examples the anode can be directly attached to the case wall. In some examples, the conductor 908 can be attached to a feedthrough. In other examples, the conductor 908 can be a feedthrough itself. This structure eliminates the need for tabs for the anodes of capacitor 900.

This application is intended to cover adaptations or variations of the present subject matter. It is to be understood that the above description is intended to be illustrative and not restrictive. The scope of the present subject matter should be determined with reference to the appended claims, along with the full scope of legal equivalents to which such claims are entitled.

## Claims

1. A method of assembling a capacitor (108; 500; 700) comprising:
sintering cathode material directly to an inside surface of a capacitor case (502; 702),
the cathode material forming one or more sintered cathodes (508; 708) having a shape;
and
placing a sintered anode (506; 706) over or around the sintered cathodes, the sintered anode having one or more mating portions (520; 720) that match the shape of the one or more sintered cathodes such that the mating portions matingly receive the sintered cathodes.

2. The method of claim 1, wherein the mating portions of the sintered anode is shaped like a fin and the sintered cathode has a fin shape.

3. The method of claim 1, wherein the sintered cathode has a cylindrical shape and the mating portions of the sintered anode includes rounded cut-outs at one or more corners of the sintered anode.

4. The method of claim 1, wherein the mating portions is conical and the sintered cathode is conical.

5. The method of any of claims 1-4, wherein the cathode includes a bed of nails structure.

6. The method of any of claims 1-5, wherein a separator is applied to the cathode,
wherein the separator includes a high dielectric polymer directly applied to an outer surface of the sintered cathode.

7. A method of assembling a capacitor (108; 600) comprising:
sintering cathode material directly to a PCB (610) within a capacitor case (502), the cathode material forming one or more sintered cathodes (508) having a shape; and placing a sintered anode (506) over the sintered cathodes, the sintered anode having one or more mating portions (520) that match the shape of the one or more sintered cathodes such that the mating portions matingly receive the sintered cathodes.

8. The method of claim 7, wherein the mating portion of the sintered anode is shaped like a fin and the sintered cathode has a fin shape.

9. The method of claim 7, wherein the sintered cathode has a cylindrical shape and the mating portions of the sintered anode includes rounded cut-outs at one or more corners of the sintered anode.

10. The method of claim 7, wherein the mating portion is conical and the sintered cathode is conical.

11. The method of any one of claims 1-10, further comprising including the capacitor in a medical system (100; 200), optionally a medical system to provide therapeutic stimulus.

12. The method of claim 11, wherein the medical system includes an implantable pacemakers, an implantable defibrillator, an implantable nerve stimulation device and devices that provide stimulation from outside the body.

13. An apparatus, including:
a capacitor case (502; 702);
cathode material sintered directly to an inside surface of the capacitor case, the cathode material forming one or more sintered cathodes (508; 708) having a shape;
a sintered anode (506; 706) placed over or around the sintered cathodes, the sintered anode having one or more mating portions (520; 720) that match the shape of the one or more sintered cathodes such that the mating portions matingly receive the sintered cathodes.

14. An apparatus including:
a capacitor case (502);
a PCB (610) within the capacitor case;
cathode material sintered directly to the PCB, the cathode material forming one or
more sintered cathodes (508) having a shape;
a sintered anode (506) placed over or around the sintered cathodes, the sintered anode having one or more mating portions (520) that match the shape of the one or more sintered cathodes such that the mating portions matingly receive the sintered cathodes.

15. The apparatus of claim 13 or claim 14, wherein the apparatus is a medical system (100; 200), optionally a medical system to provide therapeutic stimulus.

## Patentansprüche

1. Montageverfahren für einen Kondensator (108; 500; 700), umfassend:
Sintern von Kathodenmaterial direkt an eine Innenfläche eines Kondensatorgehäuses (502; 702), wobei das Kathodenmaterial eine oder mehrere gesinterte Kathoden (508; 708) mit einer Form bildet; und
Anordnen einer gesinterten Anode (506; 706) über den oder um die gesinterten Kathoden, wobei die gesinterte Anode einen oder mehrere Passabschnitte (520; 720) aufweist, die mit der Form der einen oder der mehreren gesinterten Kathoden übereinstimmen, derart dass die Passabschnitte die gesinterten Kathoden zusammenpassend aufnehmen.

2. Verfahren nach Anspruch 1, wobei die Passabschnitte der gesinterten Anode wie eine Flosse geformt sind, und die gesinterte Kathode eine Flossenform aufweist.

3. Verfahren nach Anspruch 1, wobei die gesinterte Kathode eine zylindrische Form aufweist, und die Passabschnitte der gesinterten Anode abgerundete Ausschnitte an einer oder mehreren Ecken der gesinterten Anode umfassen.

4. Verfahren nach Anspruch 1, wobei die Passabschnitte kegelförmig sind, und die gesinterte Kathode kegelförmig ist.

5. Verfahren nach einem der Ansprüche 1 bis 4, wobei die Kathode eine Nagelbettstruktur aufweist.

6. Verfahren nach einem der Ansprüche 1 bis 5, wobei ein Separator auf die Kathode aufgetragen wird, wobei der Separator ein hoch dielektrisches Polymer umfasst, das direkt auf eine Außenfläche der gesinterten Kathode aufgetragen wird.

7. Montageverfahren für einen Kondensator (108; 600), umfassend:
Sintern von Kathodenmaterial direkt an eine PCB (610) innerhalb eines Kondensatorgehäuses (502), wobei das Kathodenmaterial eine oder mehrere gesinterte Kathoden (508) mit einer Form bildet; und
Anordnen einer gesinterten Anode (506) über den gesinterten Kathoden, wobei die gesinterte Anode einen oder mehrere Passabschnitte (520) aufweist, die mit der Form der einen oder der mehreren gesinterten Kathoden übereinstimmen, derart dass die Passabschnitte die gesinterten Kathoden zusammenpassend aufnehmen.

8. Verfahren nach Anspruch 7, wobei die Passabschnitte der gesinterten Anode wie eine Flosse geformt sind, und die gesinterte Kathode eine Flossenform aufweist.

9. Verfahren nach Anspruch 7, wobei die gesinterte Kathode eine zylindrische Form aufweist, und die Passabschnitte der gesinterten Anode abgerundete Ausschnitte an einer oder mehreren Ecke der gesinterten Anode umfassen.

10. Verfahren nach Anspruch 7, wobei die Passabschnitte kegelförmig sind, und die gesinterte Kathode kegelförmig ist.

11. Verfahren nach einem der Ansprüche 1 bis 10, ferner umfassend ein Einbeziehen des Kondensators in ein medizinisches System (100; 200), gegebenenfalls ein medizinisches System zum Bereitstellen eines therapeutischen Reizes.

12. Verfahren nach Anspruch 11, wobei das medizinische System einen implantierbaren Schrittmacher, einen implantierbaren Defibrillator, eine implantierbare Nervenstimulationsvorrichtung und Vorrichtungen umfasst, die Stimulation von außerhalb des Körpers bereitstellen.

13. Vorrichtung, umfassend:
ein Kondensatorgehäuse (502; 702);
Kathodenmaterial, das direkt an eine Innenfläche des Kondensatorgehäuses gesintert ist, wobei das Kathodenmaterial eine oder mehrere gesinterte Kathoden (508; 708) mit einer Form bildet;
eine gesinterte Anode (506; 706), die über den oder um die gesinterten Kathoden angeordnet ist, wobei die gesinterte Anode einen oder mehrere Passabschnitte (520; 720) aufweist, die mit der Form der einen oder der mehreren gesinterten Kathoden übereinstimmen, derart dass die Passabschnitte die gesinterten Kathoden zusammenpassend aufnehmen.

14. Vorrichtung, umfassend:
ein Kondensatorgehäuse (502);
eine PCB (610) innerhalb des Kondensatorgehäuses;
Kathodenmaterial, das direkt an die PCB gesintert ist, wobei das Kathodenmaterial eine oder mehrere gesinterte Kathoden (508) mit einer Form bildet;
eine gesinterte Anode (506), die über den oder um die gesinterten Kathoden angeordnet ist, wobei die gesinterte Anode einen oder mehrere Passabschnitte (520) aufweist, die mit der Form der einen oder der mehreren gesinterten Kathoden übereinstimmen, derart dass die Passabschnitte die gesinterten Kathoden zusammenpassend aufnehmen.

15. Vorrichtung nach Anspruch 13 oder 14, wobei die Vorrichtung ein medizinisches System (100; 200), gegebenenfalls ein medizinisches System zum Bereitstellen eines therapeutischen Reizes ist.

## Revendications

1. Procédé d'assemblage d'un condensateur (108 ; 500 ; 700) comprenant :
le frittage d'un matériau de cathode directement sur une surface intérieure d'un boîtier de condensateur (502 ; 702), le matériau de cathode formant une ou plusieurs cathodes frittées (508 ; 708) ayant une forme ;
et
le placement d'une anode frittée (506 ; 706) sur ou autour des cathodes frittées,
l'anode frittée ayant une ou plusieurs parties d'accouplement (520 ; 720) qui correspondent à la forme de la ou des cathodes frittées de telle sorte que les parties d'accouplement reçoivent par accouplement les cathodes frittées.

2. Procédé selon la revendication 1, les parties d'accouplement de l'anode frittée ayant la forme d'une ailette et la cathode frittée ayant la forme d'une ailette.

3. Procédé selon la revendication 1, la cathode frittée ayant une forme cylindrique et les parties d'accouplement de l'anode frittée comprenant des découpes arrondies à un ou plusieurs coins de l'anode frittée.

4. Procédé selon la revendication 1, les parties d'accouplement étant coniques et la cathode frittée étant conique.

5. Procédé selon l'une quelconque des revendications 1 à 4, la cathode comprenant une structure de lit de clous.

6. Procédé selon l'une quelconque des revendications 1 à 5, un séparateur étant appliqué à la cathode, le séparateur comprenant un polymère hautement diélectrique directement appliqué à une surface extérieure de la cathode frittée.

7. Procédé d'assemblage d'un condensateur (108, 600) comprenant :
le frittage d'un matériau de cathode directement sur une PCB (610) à l'intérieur d'un boîtier de condensateur (502), le matériau de cathode formant une ou plusieurs cathodes frittées (508) ayant une forme ; et
le placement d'une anode frittée (506) sur les cathodes frittées, l'anode frittée ayant une ou plusieurs parties d'accouplement (520) qui correspondent à la forme de la ou
des cathodes frittées de telle sorte que les parties d'accouplement reçoivent par accouplement les cathodes frittées.

8. Procédé selon la revendication 7, la partie d'accouplement de l'anode frittée ayant la forme d'une ailette et la cathode frittée ayant la forme d'une ailette.

9. Procédé selon la revendication 7, la cathode frittée ayant une forme cylindrique et les parties d'accouplement de l'anode frittée comprenant des découpes arrondies à un ou plusieurs coins de l'anode frittée.

10. Procédé selon la revendication 7, la partie d'accouplement étant conique et la cathode frittée étant conique.

11. Procédé selon l'une quelconque des revendications 1 à 10, comprenant en outre l'inclusion du condensateur dans un système médical (100 ; 200), éventuellement un système médical pour fournir un stimulus thérapeutique.

12. Procédé selon la revendication 11, le système médical comprenant un stimulateur cardiaque implantable, un défibrillateur implantable, un dispositif de stimulation nerveuse implantable et des dispositifs qui fournissent une stimulation depuis l'extérieur du corps.

13. Appareil, comprenant :
un boîtier de condensateur (502 ; 702) ;
un matériau cathodique fritté directement sur une surface intérieure du boîtier de condensateur, le matériau cathodique formant une ou plusieurs cathodes frittées (508 ; 708) ayant une forme ;
une anode frittée (506 ; 706) placée sur ou autour des cathodes frittées, l'anode frittée ayant une ou plusieurs parties d'accouplement (520 ; 720) qui correspondent à la forme de la ou des cathodes frittées de telle sorte que les parties d'accouplement reçoivent par accouplement les cathodes frittées.

14. Appareil comprenant :
un boîtier de condensateur (502) ;
une PCB (610) à l'intérieur du boîtier de condensateur ;
un matériau de cathode fritté directement sur la PCB, le matériau de cathode formant une ou plusieurs cathodes frittées (508) ayant une forme ;
une anode frittée (506) placée sur ou autour des cathodes frittées, l'anode frittée ayant une ou plusieurs parties d'accouplement (520) qui correspondent à la forme de la ou des cathodes frittées de telle sorte que les parties d'accouplement reçoivent par accouplement les cathodes frittées.

15. Appareil selon la revendication 13 ou la revendication 14, l'appareil étant un système médical (100 ; 200), éventuellement un système médical pour fournir un stimulus thérapeutique.
